# EUROPÄISCHE PATENTANMELDUNG

(11) **EP 3 001 180 A1**
(43) Veröffentlichungstag der Anmeldung: **30.03.2016**
(21) Anmeldenummer: 14186890.1
(22) Anmeldetag: 29.09.2014
(51) Int. Cl.: G01N 21/39, G01J 3/433, H01S 5/062

(54) **Verfahren und Gasanalysator zur Messung der Konzentration einer Gaskomponente in einem Messgas**

(71) Anmelder: Siemens Aktiengesellschaft, 80333 München (DE)
(72) Erfinder: Steinbacher, Franz, 76137 Karlsruhe (DE)

(57) **Zusammenfassung**

Zur Messung der Konzentration einer Gaskomponente in einem Messgas (1), wird eine wellenlängenabstimmbare Laserdiode (3) mit einem Strom (i) angesteuert und das von der Laserdiode (3) erzeugte Licht (4) durch das Messgas (1) auf einen Detektor (5) geführt. Dabei wird der Strom (i) in periodisch aufeinanderfolgenden Abtastintervallen variiert, um eine interessierende Absorptionslinie der Gaskomponente wellenlängenabhängig abzutasten. Der Strom (i) kann zusätzlich im Sinne der Wellenlängenmodulationsspektroskopie mit geringer Frequenz und kleiner Amplitude sinusförmig moduliert werden. Ein von dem Detektor (5) erzeugtes Messsignal (14) wird zu einem Messergebnis (16) ausgewertet.

Um das Messsignal-Rausch-Verhältnis zu verbessern und so bei gleicher Messstrecke eine deutlich niedrigere Nachweisgrenze zu erreichen, wird der Strom (i) mit mindestens einer hohen (HF-)Frequenz im GHz-Bereich moduliert, so dass keine Wellenlängenmodulation stattfindet. Die Amplitude der HF-Modulation wird unter Ausnutzung des linearen Aussteuerbereichs der Laserdiode (3) mit maximaler Höhe gewählt. Das Messsignal (14) wird vor seiner Auswertung an der Stelle der mindestens einen hohen Frequenz demoduliert.

## Beschreibung

Die Erfindung betrifft ein Verfahren nach dem Oberbegriff des Anspruchs 1 und einen Gasanalysator nach dem Oberbegriff des Anspruchs 7.

Ein derartiges Verfahren bzw. ein derartiger Gasanalysator in Form eines Laserspektrometers sind aus der US 7 969 576 B1 bekannt.

Laserspektrometer werden insbesondere für die optische Gasanalyse in der Prozessmesstechnik eingesetzt. Dabei erzeugt eine Laserdiode Licht im Infrarotbereich, das entlang einer Messstrecke in einer Prozessanlage oder einer Gaszelle durch ein Prozessgas (Messgas) geführt und anschließend detektiert wird. Die Wellenlänge des Lichts wird auf eine spezifische Absorptionslinie der jeweils zu messenden Gaskomponente abgestimmt, wobei die Laserdiode die Absorptionslinie periodisch wellenlängenabhängig abtastet. Dazu wird die Laserdiode innerhalb von aufeinanderfolgenden Abtastintervallen mit einem rampen- oder dreieckförmigen Stromsignal angesteuert. Bei der direkten Absorptionsspektroskopie (DAS) wird die Konzentration der interessierenden Gaskomponente unmittelbar aus der an der Stelle der Absorptionslinie detektierten Minderung der Lichtintensität (Absorption) bestimmt. Bei der Wellenlängenmodulationsspektroskopie (WMS) wird während der vergleichsweise langsamen wellenlängenabhängigen Abtastung der Absorptionslinie zusätzlich die Wellenlänge des erzeugten Lichts mit einer Frequenz im kHz-Bereich und kleiner Amplitude sinusförmig moduliert. Da das Profil der Absorptionslinie nicht linear ist, werden auch Harmonische höherer Ordnung in dem Detektor- oder Messsignal erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem Messergebnis ausgewertet. Bei kleiner Modulationsamplitude ist die Detektion der n-ten Harmonischen direkt proportional zu der n-ten Ableitung des direkten Messsignals. Für den idealen Fall einer lorentzförmigen Absorptionslinie ist z. B. das 2f-Messsignal bei einem Modulationsindex von 2,2 maximal (der Modulationsindex ist das Verhältnis der spektralen Modulationsamplitude zur Breite der abgetasteten Absorptionslinie). Die weitere Auswertung kann z. B. durch Anfitten (Curve-Fitting) des im Idealfall zu erwartenden und mittels eines Näherungsmodells analytisch beschriebenen Verlaufs des demodulierten Messsignals (Sollkurve) an dessen tatsächlichen Verlauf (Istkurve) erfolgen. Da einer der Parameter des Näherungsmodells zu der Konzentration der Gaskomponente proportional ist, erhält man als Ergebnis der Auswertung, und damit als Messergebnis, die Konzentration der zu messenden Gaskomponente.

Wellenlängenmodulationsspektroskopie und Absorptionsspektroskopie haben spezifische Vor- und Nachteile. Die WMS ist insbesondere bei der Messung geringer Konzentrationen im Vorteil, weil sie Rauschen aus dem Messsignal besser ausfiltern kann. Bei höheren Konzentrationen werden aber die für die Auswertung des Messsignals notwendigen Näherungen zunehmend ungenau, wodurch der Messfehler steigt. Bei der DAS ist es umgekehrt; aufgrund der höheren Rauschempfindlichkeit ist der Messfehler bei kleinen Konzentrationen höher. Da aber keine Näherungsbeschreibung der Absorptionslinie notwendig ist, wird die Messgenauigkeit mit zunehmender Konzentration besser, weil das Nutzsignal stärker wird. Erst bei sehr hohen Konzentrationen (Sättigung der Absorption) wird das Messverfahren wieder ungenauer.

Das aus der oben genannten US 7 969 576 B1 bekannte Verfahren bzw. der Gasanalysator arbeiten auf der WMS-Basis, wobei neben der n-ten Harmonischen des Messsignals auch seine erste Harmonische, also die Grundfrequenz der Modulation, ausgewertet wird, um das Messergebnis zu normalisieren.

Die Nachweis- und Bestimmungsgrenze für die Messung der Konzentration der Gaskomponente wird durch Rauschen begrenzt, welches dem Messsignal überlagert ist und sich hauptsächlich aus dem Rauschen des Gasanalysators (Laserrauschen, Detektorrauschen) sowie dem Rauschen aus der Messstrecke (verursacht durch Turbulenzen, Partikel) zusammensetzt. Je länger die Messstrecke ist, umso größer sind die Absorption und das erhaltene Messsignal. Sollen geringe Konzentrationen gemessen werden, benötigt man eine ausreichend lange Messstrecke.

Der Erfindung liegt die Aufgabe zugrunde, das Messsignal-Rausch-Verhältnis zu verbessern und so bei gleicher Messstrecke eine deutlich niedrigere Nachweisgrenze zu erreichen.

Gemäß der Erfindung wird die Aufgabe durch das in Anspruch 1 definierte Verfahren sowie den in Anspruch 7 angegebenen Gasanalysator gelöst.

Vorteilhafte Weiterbildungen der Erfindung sind in den Unteransprüchen angegeben.

Entsprechend der Erfindung wird die Laserdiode mit einer Frequenz moduliert, die in Abhängigkeit von den Eigenschaften der Laserdiode so hoch gewählt ist, dass keine Wellenlängenmodulation des erzeugten Lichts stattfindet. Die Wellenlänge des Lichts wird über die innere Temperatur der Laserdiode eingestellt bzw. verändert, wobei diese innere Temperatur wiederum über die Verlustleistung durch den Laserstrom und über die Umgebungstemperatur einstellbar bzw. veränderbar ist. Eine Wellenlängenmodulation kann daher nur mit niedrigen Modulationsfrequenzen, maximal im kHz-Bereich, erfolgen. Bei höheren Frequenzen wird dagegen nur noch die Intensität des Lichts, nicht aber seine Wellenlänge moduliert. So endet bei den derzeit erhältlichen VCSE-Lasern die Wellenlängenmodulation bei einigen MHz.

Durch die erfindungsgemäße hochfrequente (HF-)Modulation wird das Basisband des auszuwertenden Messsignals aus dem durch das Rauschen des Gasanalysators und der Messstrecke gestörten Frequenzbereich nahe DC in einen hohen Frequenzbereich kopiert, in dem dieses Rauschen nicht mehr vorhanden ist. Das bei der Frequenz der HF-Modulation demodulierte Messsignal enthält demnach dieselben analytischen Informationen (Nutzinformationen) wie das aus der herkömmlichen Ansteuerung der Laserdiode ohne die erfindungsgemäße HF-Modulation resultierende Messsignal, und zwar unabhängig davon, ob lediglich eine rampen- oder dreieckförmige Variation des Stroms der Laserdiode zum Zwecke der wellenlängenabhängigen Abtastung der interessierenden Absorptionslinie und zur Auswertung des Messsignals auf Basis der direkten Absorptionsspektroskopie (DAS) oder zusätzlich eine NF-Modulation des Stromes zum Zwecke einer WMS-Auswertung des Messsignals erfolgt. Das bei der Frequenz der HF-Modulation demodulierte Messsignal kann daher auf dieselbe Weise wie das bisher verwendete Messsignal an dessen Stelle oder ergänzend zu diesem ausgewertet werden.

Bei der wellenlängenabhängigen Abtastung der interessierenden Absorptionslinie der Gaskomponente durch die periodische Variation des Stroms der Laserdiode ändert sich der aus der zusätzlichen HF-Modulation der Laserdiode resultierende Signalanteil des Messsignals prozentual in demselben Maße wie der extrem niederfrequente Signalanteil (nahezu Gleichanteil) des Messsignals, welcher sich aus der Ansteuerung der Laserdiode mit dem rampen- oder dreieckförmigen Stromsignal ergibt. Beide Signalanteile können daher einzeln oder zusammen, z. B. nach Addition, gemäß der DAS ausgewertet werden. Dies gilt in analoger Weise auch für die Auswertung des Messsignals auf WMS-Basis. Da bei der HF-Modulation der Laserdiode keine Wellenlängenmodulation des Lichts stattfindet, die die WMS-Auswertung stören würde, kann die Amplitude der HF-Modulation so hoch gewählt werden, wie es der lineare Aussteuerbereich der Laserdiode zulässt. In jedem Fall ist die Amplitude der HF-Modulation um ein Vielfaches höher, als die der niederfrequenten WMS-Modulation, wo der Modulationsindex in Abhängigkeit von der Breite der abzutastenden Absorptionslinie zu wählen ist.

Die hinsichtlich der Auswertung des Messsignals unterschiedlichen Messungen auf Basis von DAS und WMS können gleichzeitig in jedem Abtastintervall oder abwechselnd in aufeinanderfolgenden Abtastintervallen erfolgen und ihre Ergebnisse z. B. durch Mittelwertbildung zu dem Messergebnis verknüpft werden.

Die HF-Modulation kann mit nur einer, in vorteilhafter Weise aber auch mit mehreren oder vielen Frequenzen erfolgen, wobei die bei diesen Frequenzen demodulierten Messsignale entweder einzeln auf der Basis von WMS und/oder DAS ausgewertet und danach zu dem Messergebnis beispielsweise durch Datenfusion (Data Fusion, Multi-Sensor Data Fusion) zusammengeführt, im einfachsten Fall addiert, werden oder zuerst zusammengeführt und dann ausgewertet werden. Durch die Zusammenführung der bei mehreren oder vielen Frequenzen demodulierten Messsignale bzw. der Ergebnisse ihrer Einzelauswertungen erhält man entsprechend viel auswertbare Signalenergie. Der Abstand zwischen den für die HF-Modulation verwendeten Frequenzen muss dabei so groß sein, dass sich die auszuwertenden Frequenzbänder nicht überlappen und darüber hinaus durch Bandpassfilterung sauber erfasst werden können. Da im Unterschied zu den enthaltenen Nutzinformationen das Rauschen in den verschiedenen Frequenzbändern nicht korreliert, wird der Signal-Rauschabstand verbessert. Die Amplituden der sich überlagernden HF-Modulationen werden so gewählt, dass die Laserdiode innerhalb ihres linearen Aussteuerbereichs soweit wie möglich ausgesteuert wird.

Die Messung auf Basis von WMS kann in herkömmlicher Weise erfolgen, indem der Strom der Laserdiode und damit die Wellenlänge des erzeugten Lichts mit einer zusätzlichen Frequenz moduliert wird und das Messsignal im Basisband und das demodulierte Messsignal aus dem höheren Frequenzbereich bei einer Harmonischen, insbesondere der zweiten Harmonischen, der zusätzlichen Frequenz ausgewertet werden.

Die WMS kann aber auch modifiziert und erweitert werden, so wie dies z. B. Gegenstand der älteren und noch nicht veröffentlichten deutschen Patentanmeldung mit dem Aktenzeichen DE102014215848.6 ist. Dort erfolgt die niederfrequente WMS-Modulation mit mehreren zusätzlichen Frequenzen, die um den doppelten Betrag der niedrigsten zusätzlichen Frequenz voneinander beabstandet sind, also beispielsweise f_{NF}, 3f_{NF}, 5f_{NF} und 7f_{NF}. Die WMS-Auswertung des Messsignals bzw. des HF-demodulierten Messsignals erfolgt an den Stellen der zweiten Harmonischen dieser Frequenzen, also 2f_{NF}, 6f_{NF}, 10f_{NF} und 14f_{NF}, und mindestens einer ihrer Summen- und Differenzfrequenzen, also 2f_{NF}, 4f_{NF}, 6f_{NF}, 8f_{NF}, 6f_{NF}, ... 14f_{NF}. Wegen der nichtlinearen Form der Absorptionslinie enthält das Messsignal nicht nur die Vielfachen (Harmonischen) der bei der Modulation verwendeten Frequenzen, sondern auch die Summen- und Differenzen dieser Frequenzen. Da die verwendeten WMS-Modulationsfrequenzen um den doppelten Betrag 2f_{NF} der kleinsten Modulationsfrequenz f_{NF} auseinander liegen, fallen ihre Summen- und Differenzfrequenzen entweder mit den zweiten Harmonischen zusammen oder liegen genau in der Mitte zwischen diesen. Daher liegen auch die Frequenzanteile des Messsignals jeweils um den doppelten Betrag 2f_{NF} der kleinsten Modulationsfrequenz f_{NF} auseinander. Die entsprechenden Messsignalanteile weisen jeweils die gleichen Verläufe auf, so dass sie sich in konstruktiver Weise überlagern und es gelingt, entsprechend viel auswertbare Signalenergie aus der Absorption zu erhalten. Da das Rauschen bei den unterschiedlichen Frequenzen im Unterschied zu den sich addierenden Signalanteilen nicht korreliert ist, entsteht bei der Auswertung zu dem Messergebnis ein sehr hoher Signal-Rauschabstand.

Zusätzlich oder ergänzend kann das Messsignal bzw. demodulierte Messsignal bei den Modulationsfrequenzen f_{NF}, 3f_{NF}, 5f_{NF} und 7f_{NF} ausgewertet werden.

Zur weiteren Erläuterung der Erfindung wird im Folgenden auf die Figuren der Zeichnung Bezug genommen; im Einzelnen zeigen:
- Figur 1: ein erstes Ausführungsbeispiel des erfindungsgemäßen Gasanalysators,
- Figur 2: ein Beispiel einer HF-Demodulation des Messsignals, und

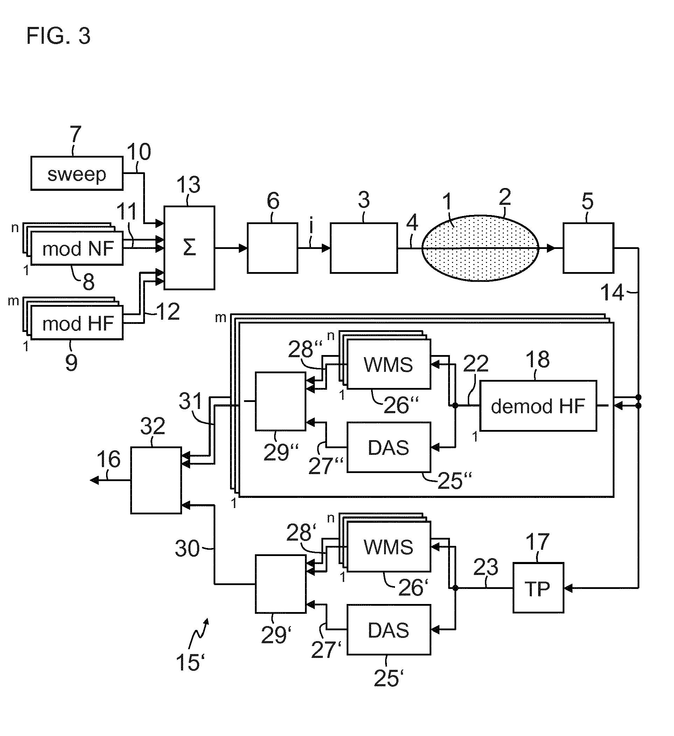

- Figur 3: ein weiteres Ausführungsbeispiel des erfindungsgemäßen Gasanalysators.

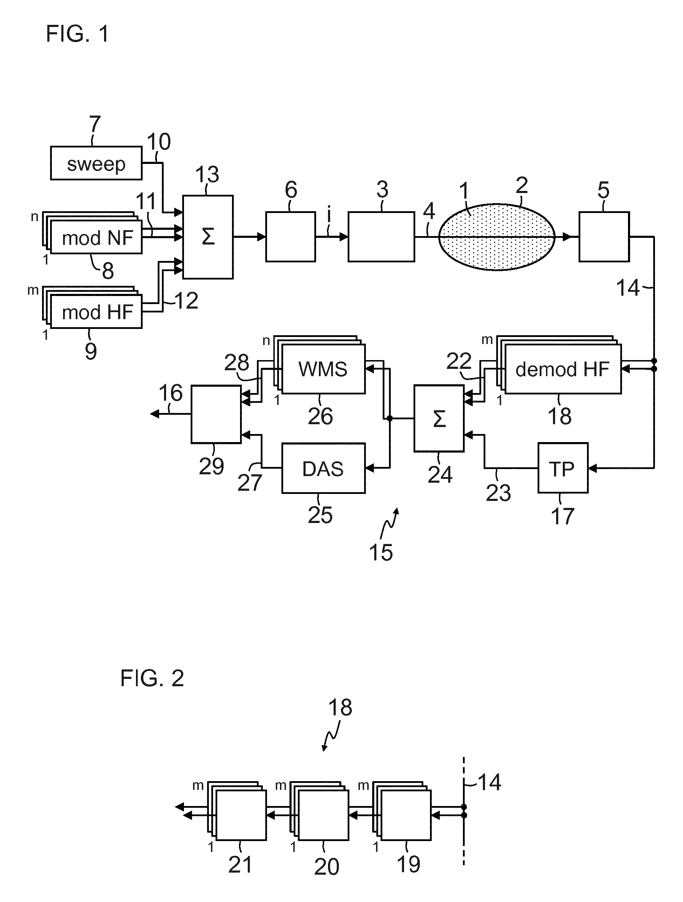

Figur 1 zeigt, in Form eines stark vereinfachten Blockschaltbildes, einen Gasanalysator zur Messung der Konzentration mindestens einer interessierenden Gaskomponente eines Messgases 1, das in einem Messvolumen 2, beispielsweise einer Gaszelle oder einer Prozessgasleitung, enthalten ist. Der Gasanalysator enthält eine Laserdiode 3, deren Licht 4 nach Durchstrahlen des Messgases 1 auf einen Detektor 5 fällt. Eine steuerbare Stromquelle 6 speist die Laserdiode 3 mit einem Injektionsstrom i, wobei die Intensität und die Wellenlänge des erzeugten Lichts 4 von dem Strom i und der Betriebstemperatur der Laserdiode 3 abhängen. Zur Variation und Modulation des Stromes i erzeugen ein Signalgenerator 7, eine Niederfrequenz-(NF-)Modulationseinrichtung 8 und eine Hochfrequenz-(HF-)Modulationseinrichtung 9 unterschiedliche Signale 10, 11, 12, die der Stromquelle 6 über einen Summierer 13 zugeführt werden.

Mit dem Signal 10 des Signalgenerators 7 wird der Strom i periodisch entsprechend einer vorgegebenen, vorzugsweise rampen- oder dreieckförmigen Funktion variiert, um mit der mehr oder weniger linear folgenden Wellenlänge des erzeugten Lichts 4 eine ausgewählte Absorptionslinie der interessierenden Gaskomponente abzutasten. Das Signal 10 kann zusätzlich Bursts enthalten, die in regelmäßigen Abständen, z. B. nach jeder Abtastperiode, aufeinander folgen und später eine Normalisierung der Messung ermöglichen.

Die Niederfrequenz-(NF-)Modulationseinrichtung 8 ist vorhanden, wenn die Konzentration der interessierenden Gaskomponente auf Basis der Wellenlängenmodulationsspektroskopie (WMS) erfolgen soll. In diesem Fall wird der Strom i und damit die Wellenlänge des erzeugten Lichts 4 mit einer Frequenz im kHz-Bereich und kleiner Amplitude sinusförmig moduliert. Wie angedeutet ist, kann die NF-Modulation auf n verschiedene Frequenzen erweitert werden.

Die Hochfrequenz-(HF-)Modulationseinrichtung 8 dient dazu, den Strom i mit einer sehr hohen Frequenz im MHz-Bereich, z. B: 50 MHz, und hoher Amplitude zu modulieren. Die Hochfrequenz ist dabei in Abhängigkeit von den Eigenschaften der verwendeten Laserdiode 3 so gewählt, dass nur die Intensität des erzeugten Lichts 4 moduliert wird und keine Wellenlängenmodulation stattfindet. Die Amplitude der Modulation ist im Rahmen des linearen Aussteuerbereichs der Laserdiode 3 maximal; sie kann beispielsweise im Größenbereich des halben Mittelwerts der Stromrampe (Signal 10) liegen. Wie die NF-Modulation kann auch die HF-Modulation auf mehrere oder viele, hier m, verschiedene Frequenzen erweitert werden. Der Abstand zwischen den Frequenzen muss dabei so groß sein, dass sich die auf WMS-Basis auszuwertenden Frequenzbänder nicht überlappen und nach Detektion durch Bandpassfilterung sauber erfasst werden können.

Der Detektor 5 erzeugt in Abhängigkeit von der detektierten Lichtintensität ein Messsignal 14, das anhand der aus den Bursts des Signals 10 resultierenden Signalanteile automatisch verstärkt und normalisiert werden kann. Im Weiteren wird das Messsignal 14 in einer Auswerteeinrichtung 15 zu einem die Konzentration der interessierenden Gaskomponente in dem Messgas 1 angebenden Messergebnis 16 verarbeitet. Aufgrund der HF-Modulation der Wellenlänge des Lichts 4 wird das Basisband des Messsignals 14 mit den darin enthaltenen Nutzinformationen in höhere Frequenzbereiche kopiert. Die in dem Basisband enthaltenen Nutzinformationen werden durch Filtern des Messsignals 14 in einem Tiefpassfilter 17 mit einer unter der niedrigsten der verwendeten HF-Modulationsfrequenz liegenden Grenzfrequenz erhalten. Um die Nutzinformationen aus den höheren Frequenzbändern zu erhalten, wird das Messsignal 14 parallel zur Tiefpassfilterung in einer HF-Demodulationseinrichtung 18 bei jeder der m (m ≥ 1) verwendeten Frequenzen der HF-Modulation demoduliert.

Wie Figur 2 zeigt, erfolgt die Demodulation in m parallelen Kanälen, die jeweils ein Bandpassfilter 19 und einen Lock-in Verstärker 20 mit nachgeordnetem Tiefpassfilter 21 aufweisen. Dabei wird das bandpassgefilterte Messsignal 14 durch Multiplikation mit einem Referenzsignal bei der jeweiligen HF-Demodulationsfrequenz phasensensitiv demoduliert, und durch die anschließende Tiefpassfilterung die Inphasenkomponente, also der Nutzsignalanteil 22 des demodulierten Messsignals 14 extrahiert. Die Bandbreite des Bandpassfilters 19 und die Grenzfrequenz des Tiefpassfilters 21 sind so groß, dass sie die auf DAS- und/oder WMS-Basis auszuwertenden Frequenzbänder des Messsignals 14 durchlassen.

Zurück zu Figur 1 werden die durch die Tiefpassfilterung und Demodulation erhaltenen Nutzinformationen 23, 22 in einem Summierer 24 addiert und anschließend in einer Recheneinrichtung 25 auf DAS-Basis und/oder in einer weiteren Recheneinrichtung 26 auf WMS-Basis ausgewertet. Die Ergebnisse 27, 28 der DAS- und WMS-Auswertungen werden in einer dritten Recheneinrichtung 29 addiert oder unter Zuhilfenahme der statistischen Signalverarbeitung zusammengeführt, um schließlich als Messergebnis 16 die Konzentration der zu messenden Gaskomponente zu erhalten.

Die WMS-Auswertung erfolgt z. B. an den Stellen der zweiten Harmonischen der n (n ≥ 1) verwendeten Frequenzen der niederfrequenten WMS-Modulation. In diesem Fall handelt es sich um n Einzelauswertungen, die z. B. n Kurvenverläufe ergeben. Darüber hinaus kann die WMS-Auswertung auch an den Stellen der Grundfrequenzen oder weiterer höherer Harmonischer erfolgen, was zu zusätzlichen Sätzen von jeweils n Einzelauswertungen führt. Die n Kurvenverläufe innerhalb jedes einzelnen Satzes korrelieren und können zu einem Zwischenergebnis ausgewertet werden. Die Zwischenergebnisse aus den unterschiedlichen Sätzen werden dann ihrerseits zusammengeführt, im einfachsten Fall addiert.

Die WMS-Auswertung kann erweitert werden, indem die niederfrequente WMS-Modulation bei Frequenzen erfolgt, die um den doppelten Betrag der niedrigsten zusätzlichen Frequenz voneinander beabstandet sind, also für n = 4 die Frequenzen: f_{NF}, 3f_{NF}, 5f_{NF} und 7f_{NF}. Die WMS-Auswertung kann dann sowohl an den Stellen der zweiten Harmonischen dieser Frequenzen, also 2f_{NF}, 6f_{NF}, 10f_{NF} und 14f_{NF}, als auch an den Stellen ihrer Summen- und Differenzfrequenzen, also 2f_{NF}, 4f_{NF}, 6f_{NF}, 8f_{NF}, 6f_{NF}, ... 14f_{NF}, erfolgen. In diesem Fall handelt es sich um 2(2n-1) = 14 Einzelauswertungen, die 14 Kurvenverläufe ergeben. Wie oben bereits erwähnt, enthält das Messsignal 14 wegen der nichtlinearen Form der abgetasteten Absorptionslinie nicht nur die zweiten Harmonischen der bei der Modulation verwendeten Frequenzen, sondern auch die Summen- und Differenzen dieser Frequenzen, die entweder mit den zweiten Harmonischen zusammenfallen oder genau in der Mitte zwischen diesen liegen. Die Messsignalanteile an den Stellen der Frequenzen 2f_{NF}, 4f_{NF}, 6f_{NF}, 8f_{NF}, 6f_{NF}, ... 14f_{NF} weisen jeweils die gleichen Verläufe auf, so dass sie sich konstruktiv überlagern und so die auswertbare Signalenergie aus der Absorption erhöhen.

Figur 2 zeigt ein weiteres Ausführungsbeispiel des erfindungsgemäßen Gasanalysators mit einer im Vergleich zu Figur 1 modifizierten Auswerteeinrichtung 15'. Die durch Filtern des Messsignals 14 mittels des Tiefpasses 17 sowie durch Demodulation an den Stellen der m verschiedenen Frequenzen der HF-Modulation enthaltenen Nutzinformationen 23, 22 werden nicht, wie im Falle des Ausführungsbeispiels nach Figur 1, addiert und anschließend ausgewertet, sondern einzeln für den Tiefpasskanal und jeden der m HF-Demodulationskanäle in Recheneinrichtungen 25', 25'' auf DAS-Basis und weiteren Recheneinrichtungen 26', 26'' auf WMS-Basis ausgewertet. Zum Schluss werden die Ergebnisse 30, 31 aus den unterschiedlichen in einer Recheneinheit 32, beispielsweise einem Addierer zu dem Messergebnis 16 zusammengeführt.

## Patentansprüche

1. Verfahren zur Messung der Konzentration einer Gaskomponente in einem Messgas (1) mittels eines Gasanalysators, wobei
- eine wellenlängenabstimmbare Laserdiode (3) mit einem Strom (i) angesteuert und das von der Laserdiode (3) erzeugte Licht (4) durch das Messgas (1) auf einen Detektor (5) geführt wird,
- zur wellenlängenabhängigen Abtastung einer interessierenden Absorptionslinie der Gaskomponente der Strom (i) in periodisch aufeinanderfolgenden Abtastintervallen variiert wird,
- der Strom (i) zusätzlich mit vorgegebener Frequenz und Amplitude sinusförmig moduliert wird und
- ein von dem Detektor (5) erzeugtes Messsignal (14) bei der Frequenz der Modulation demoduliert und das dabei erhaltene demodulierte Messsignal (22) zur Erzeugung eines Messergebnisses (16) ausgewertet wird,
**dadurch gekennzeichnet,**
- **dass** die Frequenz der Modulation in Abhängigkeit von den Eigenschaften der Laserdiode (3) so hoch gewählt ist, dass keine Wellenlängenmodulation stattfindet und
- **dass** die Amplitude der Modulation innerhalb und unter Ausnutzung des linearen Aussteuerbereichs der Laserdiode (3) mit maximaler Höhe gewählt ist.

2. Verfahren nach Anspruch 1, **dadurch gekennzeichnet,**
- **dass** der Strom (i) der Laserdiode (3) mit mindestens einer weiteren Frequenz moduliert wird, die ebenfalls so hoch gewählt ist, dass keine Wellenlängenmodulation stattfindet,
- **dass** die Amplituden der Modulationen innerhalb und unter Ausnutzung des linearen Aussteuerbereichs der Laserdiode (3) mit maximaler Höhe gewählt sind,
- **dass** das von dem Detektor (5) erzeugte Messsignal (14) zusätzlich bei der Frequenz der mindestens einen weiteren Modulation demoduliert wird und
- **dass** die erhaltenen demodulierten Messsignale (22) entweder einzeln ausgewertet und anschließend zu dem Messergebnis (16) zusammengeführt oder zuerst zusammengeführt und anschließend zu dem Messergebnis (16) ausgewertet werden.

3. Verfahren nach Anspruch 1 oder 2, basierend auf der direkten Absorptionsspektroskopie.

4. Verfahren nach einem der vorangehenden Ansprüche basierend auf der Wellenlängenmodulationsspektroskopie wobei der Strom (i) der Laserdiode (3) zusätzlich mit geringer Amplitude und mindestens einer zusätzlichen Frequenz moduliert wird, die so niedrig gewählt ist, dass eine Wellenlängenmodulation stattfindet.

5. Verfahren nach Anspruch 3 und 4, **dadurch gekennzeichnet, dass** Messungen auf Basis der direkten Absorptionsspektroskopie und der Wellenlängenmodulationsspektroskopie gleichzeitig in jedem Abtastintervall oder abwechselnd in aufeinanderfolgenden Abtastintervallen erfolgen und ihre Ergebnisse durch Mittelwertbildung zu dem Messergebnis verknüpft werden.

6. Verfahren nach Anspruch 4 oder 5, **dadurch gekennzeichnet, dass** bei Modulation mit mehreren zusätzlichen Frequenzen diese um den doppelten Betrag der niedrigsten zusätzlichen Frequenz voneinander beabstandet sind und dass die auf der Wellenlängenmodulationsspektroskopie basierende Auswertung des demodulierten Messsignals an den Stellen der zweiten Harmonischen der zusätzlichen Frequenzen und mindestens einer der Summen- und Differenzfrequenzen der zusätzlichen Frequenzen erfolgt.

7. Gasanalysator zur Messung der Konzentration einer Gaskomponente in einem Messgas (1) mit
- einer wellenlängenabstimmbaren Laserdiode (3),
- einer die Laserdiode (3) mit einem Strom (i) speisenden Stromquelle (6),
- einem Signalgenerator (7), der die Stromquelle (6) steuert um den Strom (i) zur wellenlängenabhängigen Abtastung einer interessierenden Absorptionslinie der Gaskomponente in periodisch aufeinanderfolgenden Abtastintervallen zu variieren,
- einer Modulationseinrichtung (9), die die Stromquelle (6) steuert um den Strom (i) mit vorgegebener Frequenz und Amplitude sinusförmig zu modulieren,
- Mitteln, die das modulierte Licht (4) durch das Messgas (1) auf einen Detektor (5) führen, und
- einer Auswerteeinrichtung (15, 15'), die ein von dem Detektor (5) erzeugtes Messsignal (14) bei der Frequenz der Modulation demoduliert und das dabei erhaltene demodulierte Messsignal (22) zur Erzeugung eines Messergebnisses (16) auswertet,
**dadurch gekennzeichnet,**
- **dass** die Modulationseinrichtung (9) dazu ausgebildet ist, die Modulation mit einer so hohen Frequenz durchzuführen, dass keine Wellenlängenmodulation stattfindet, und
- **dass** die Modulationseinrichtung (9) ferner dazu ausgebildet ist, die Modulation mit einer innerhalb des linearen Aussteuerbereichs liegenden und diesen maximal ausnutzenden Amplitude durchzuführen.

8. Gasanalysator nach Anspruch 7, **dadurch gekennzeichnet,**
- **dass** die Modulationseinrichtung (9) ferner dazu ausgebildet ist, den Strom (i) der Laserdiode (3) mit mindestens einer weiteren Frequenz zu modulieren, die ebenfalls so hoch gewählt ist, dass keine Wellenlängenmodulation stattfindet,
- **dass** die Amplituden der Modulationen innerhalb und unter Ausnutzung des linearen Aussteuerbereichs der Laserdiode (3) mit maximaler Höhe gewählt sind,
- **dass** die Auswerteeinrichtung (15, 15') dazu ausgebildet ist, das von dem Detektor (5) erzeugte Messsignal (14) zusätzlich bei der Frequenz der mindestens einen weiteren Modulation zu demodulieren und die erhaltenen demodulierten Messsignale (22) entweder einzeln auszuwerten und anschließend zu dem Messergebnis (16) zusammenzuführen oder zuerst zusammenzuführen und anschließend zu dem Messergebnis (16) auszuwerten.

9. Gasanalysator nach Anspruch 7 oder 8, **dadurch gekennzeichnet, dass** die Auswerteeinrichtung (15, 15') dazu ausgebildet ist, das demodulierte Messsignal (22) auf der Grundlage der direkten Absorptionsspektroskopie auszuwerten.

10. Gasanalysator nach Anspruch 7, 8 oder 9, **dadurch gekennzeichnet,**
- **dass** eine Niederfrequenz-(NF-)Modulationseinrichtung (8) vorhanden ist, die die Stromquelle (6) steuert um den Strom (i) zusätzlich mit geringer Amplitude und mindestens einer zusätzlichen Frequenz zu modulieren, die so niedrig gewählt ist, dass eine Wellenlängenmodulation stattfindet, und
- **dass** die Auswerteeinrichtung (15, 15') dazu ausgebildet ist, das demodulierte Messsignal (14) auf der Grundlage der Wellenlängenmodulationsspektroskopie auszuwerten.
